# EUROPÄISCHE PATENTANMELDUNG

(11) **EP 4 802 997 A1**
(43) Veröffentlichungstag der Anmeldung: **09.09.2026**
(21) Anmeldenummer: 25214297.1
(22) Anmeldetag: 07.11.2025
(51) Int. Cl.: A61B 5/087, A61B 7/00, A63B 23/18

(54) **VERFAHREN ZUR ERFASSUNG AN EINEM ATEMÜBUNGSGERÄT DURCHGEFÜHRTER ATEMÜBUNGEN**

(30) Priorität: 05.03.2025 DE 102025108401
(71) Anmelder: CEGLA Medizintechnik GmbH, 56410 Montabaur (DE)
(72) Erfinder: Ebinger, Andrea, 56410 Montabaur (DE)
(74) Vertreter: Christ, Niko

(57) **Zusammenfassung**

Zur Durchführung von Atemübungen mit einem Atemübungsgerät ist vorgesehen, dass das Atemübungsgerät aus einem Gehäuse mit einem Strömungskanal besteht, in dem ein der Intensität und/oder dem Schwingfrequenzverlauf und/oder der Dauer und/oder der Amplitude nach mithilfe eines Datenverarbeitungsgerätes erfassbares, durch die Atemluft eines Anwenders beeinflusstes Strömungsgeräusch erzeugt wird. Dieses Strömungsgeräusch wird durch ein Mikrofon erfasst und an eine Auswerteeinheit weitergeleitet, welche es hinsichtlich der genannten Parameter analysiert. Das System gibt dem Anwender Echtzeit-Rückmeldungen zur Atemtechnik und ermöglicht individuelle Anpassungen der Therapie. Es umfasst auch Funktionen zur Erfassung von Atemmustern, Ruhephasen und der Einstellung einer variablen oder statischen Stenose im Strömungskanal. Das Verfahren nutzt elektronische Kommunikationsmittel und kann in dem Atemübungsgerät, oder auch in einem Smartphone oder Wearable oder anderen Geräten integriert werden. Es bietet eine präzise Überwachung und Auswertung der Atemübungen, um die Effektivität der Übungen zu maximieren.

## Beschreibung

Die vorliegende Erfindung betrifft ein Verfahren zur Erfassung an einem Atemübungsgerät durchgeführter Atemübungen, wobei das Atemübungsgerät einen Strömungskanal ausbildet, in dem während der Atemübungen durch eine hierbei eingebrachte Luftströmung ein Strömungsgeräusch erzeugt wird, welches mithilfe eines Mikrofons erfasst und von einer Auswerteeinheit ausgewertet wird.

Ein solches Atemübungsgerät ist bereits aus der EP 4 175 703 B1 vorbekannt. Dort ist ein Atemübungsgerät zur Behandlung der Atemmuskulatur, Lungenfunktionen und zur Schleimlösung in den Atemwegen, Lungen und/oder im Nasen- und Rachenraum eines Anwenders beschrieben. Das Gerät besteht aus einem Hohlkörper mit einem Mund- oder Nasenstück und mindestens einem Schwingkörper im Inneren, der durch die Ein- und Ausatmung des Anwenders in Vibration versetzt wird. Diese Vibration erzeugt oszillierende Luftdruckschwankungen, die zur Erweiterung der Bronchien und zur Schleimlösung beitragen. Das Gerät ermöglicht individuelle Einstellungen der Luftströmungen und Widerstände, um eine anwenderspezifische Therapie zu gewährleisten.

Auf dem Gebiet der Atemübungen und im Bereich der Atemwegstherapie ist es üblich, Geräte zu verwenden, welche die Anwender bei der Durchführung von Atemübungen unterstützen. Diese Geräte sind darauf ausgelegt, die Atemmuskulatur zu stärken, die Lungenkapazität zu erhöhen und die allgemeine Atemfunktion zu verbessern. Auch ist es bekannt, Geräte bereitzustellen, welche die Nutzung medizinischer Geräte nachempfinden um einen Trainingseffekt auch hinsichtlich der Anwendung solcher Geräte zu erzielen. Bekannte Systeme umfassen typischerweise mechanische Vorrichtungen, die durch den Atemfluss des Anwenders aktiviert werden und visuelle oder akustische Rückmeldungen geben, um den Anwender bei der korrekten Durchführung der Übungen zu unterstützen. Solche Geräte sind oft einfach konstruiert und bieten grundlegende Funktionen wie die Messung des Atemvolumens oder der Atemfrequenz.

Die GB 2 563 033 A betrifft eine Messvorrichtung zur Ermittlung des Luftstroms während der Inspiration und/oder Exspiration. Das Gerät verwendet eine Vielzahl von Riffelungen, die in den Luftstrompfad hineinragen, um mindestens zwei Schallsignale bei unterschiedlichen Resonanzen zu erzeugen. Diese Riffelungen können in einem Kunststoffkörper integriert sein und ermöglichen die Anzeige der Luftströmungsrate ohne bewegliche Teile oder die Notwendigkeit einer regelmäßigen Kalibrierung. Das Gerät kann als Spirometer verwendet werden und bietet die Möglichkeit, die erzeugten Schallsignale über eine mobile App zu analysieren, um Gesundheitsfeedback zu geben oder Warnungen auszulösen. Es ist besonders nützlich für die Überwachung von Atemwegserkrankungen wie Asthma, da es dem Benutzer ermöglicht, auf Veränderungen in der Atemkapazität zu reagieren.

Ferner ist auf diesem Gebiet die EP 3 586 740 A1 bekannt, gemäß der eine Lungenfunktionsvorrichtung, wie etwa ein Spirometer, eingesetzt wird, um durch eine Differenzmessung während der Inspiration und der Exspiration, festzustellen, wie viel von einem betrachteten Stoff in der Lunge eines Anwenders zurückbleibt. Hierbei können zusätzliche Erkenntnisse über Vitalitätsparameter des betreffenden Anwender gewonnen werden, wenn mit dem Gerät vorgegebene Atemübungen durchgeführt werden, die durch ein zugeordnetes Datenverarbeitungsgerät, wie etwa ein Smartphone, angeleitet werden. Auch können dort Umgebungsbedingungen berücksichtigt werden, die das Datenverarbeitungsgerät selbst ermittelt oder aus bereitgestellten Quellen einbezieht. Anhand der so ermittelten Daten kann das System Gesundheitsdaten des Anwenders erstellen und ermitteln, aber auch Handlungsempfehlungen bereitstellen, die dem Anwender oder behandelnden Personen bereitgestellt werden können. Die gewonnenen Daten können zur Verbesserung der Diagnose und Therapie von Atemwegserkrankungen wie Asthma oder einer chronisch obstruktiven Lungenerkrankung genutzt werden.

Das Dokument US 10,665,132 B2 beschreibt darüber hinaus ein Trainingsgerät zur Schulung der korrekten Anwendung eines druckbeaufschlagten Dosierinhalators. Das Gerät besteht aus einem Gehäuse, einem Luftausstoßmittel wie etwa einem Blasebalg zur Luftausstoßung entlang eines Luftstrompfades und einem Betätigungsmittel zur Betätigung des Luftausstoßmittels. Der Luftstrompfad sieht vor, dass das Luftausstoßmittel beim Ausstoß von Luft aufgrund einer Einschnürung ein akustisches Signal erzeugt, welches das Geräusch bei der Verwendung eines Dosierinhalators imitiert. Das Gerät zielt darauf ab, kostengünstig und für den Heimgebrauch geeignet zu sein, ohne Treibgase einzusetzen, und bietet eine Anleitung zur Koordination von Inhalation und Betätigung des Medikamentenbehälters. Es umfasst mehrere Merkmale, um eine realistische Trainingserfahrung zu bieten, einschließlich eines Zählers zur Anzeige der verstrichenen Zeit seit der Betätigung des Luftausstoßmittels.

Gemäß bekannter Technik werden Atemübungsgeräte also häufig in Kombination mit manuellen oder elektronischen Auswertungen verwendet, um die Effektivität der Atemübungen zu überwachen. Diese Systeme können jedoch in ihrer Genauigkeit und Benutzerfreundlichkeit eingeschränkt sein. Beispielsweise erfassen einige Geräte nur grobe Daten, die eine detaillierte Analyse der Atemmuster des Anwenders erschweren. Andere Systeme erfordern eine manuelle Eingabe von Daten oder eine separate Auswertung durch medizinisches Fachpersonal, was den Prozess zeitaufwändig und fehleranfällig machen kann.

Vor diesem Hintergrund liegt der vorliegenden Erfindung die Aufgabe zu Grunde, ein Verfahren zu schaffen, bei dem mithilfe eines einfachen und für den Anwender leicht bedienbaren Atemübungsgeräts ein individuelles Training oral, tracheal und/oder nasal durchführbar ist, welches über eine Auswerteeinheit sowohl anleitbar als auch überwachbar ist und aufgrund einer Rückmeldung auswertbar und aufgrund weiterer Umgebungsparameter anpassbar ist.

Gelöst wird diese Aufgabe durch ein Verfahren gemäß den Merkmalen des unabhängigen Anspruchs 1. Sinnvolle Ausgestaltungen eines solchen Verfahrens können den sich hieran anschließenden abhängigen Ansprüchen entnommen werden.

Insoweit vorgesehen ist ein Verfahren zur Erfassung an einem Atemübungsgerät durchgeführter Atemübungen, wobei das Atemübungsgerät einen Strömungskanal ausbildet, in dem während der Atemübungen durch eine hierbei eingebrachte Luftströmung ein Strömungsgeräusch erzeugt wird, welches mithilfe eines Mikrofons erfasst und von einer Auswerteeinheit ausgewertet wird. Ein solches Verfahren ist erfindungsgemäß dadurch gekennzeichnet, dass die Auswerteeinheit während der Atemübung ein Atemmuster vorgibt und Intensität und/oder Frequenzverlauf und/oder Dauer und/oder Amplitude des erfassten Strömungsgeräuschs auswertet und, vorzugsweise im Zuge einer Adhärenzmessung, mit dem vorgegebenen Atemmuster vergleicht und mithilfe von Ausgabemitteln der Auswerteeinheit eine Rückmeldung ausgibt.

Mit anderen Worten wird ein Verfahren zur Durchführung von Atemübungen beschrieben, bei dem ein Atemübungsgerät verwendet wird, das über ein Gehäuse verfügt, das wenigstens einen Strömungskanal zwischen einer Atemöffnung und einer Stenose bildet. Bedingt durch die Atmung eines Anwenders in das Atemübungsgerät hinein, stellt sich durch die hierdurch hervorgerufene Luftströmung ein Strömungsgeräusch ein, das erfasst und mithilfe einer Auswerteeinheit ausgewertet wird.

Die Auswerteeinheit ermittelt während der Atmung des Anwenders Intensität und/oder Schwingfrequenzverlauf und/oder Dauer und/oder Amplitude des Strömungsgeräusches. Sie zeichnet diese Parameter während einer Atemübung auf und analysiert und vergleicht sie mit einer für den Anwender ausgegebenen Vorgabe eines Atemmusters. Über seitens der Auswerteeinheit bereitgehaltene Ausgabemittel erhält der Anwender eine Rückmeldung zu diesen Parametern und/oder eine Rückmeldung zur Übereinstimmung der Atemübung mit dem vorgegebenen Atemmuster, vorzugsweise im Rahmen einer Adhärenzmessung.

Ein Vorteil dieses Verfahrens liegt in der präzisen Erfassung und Auswertung der Atemparameter (in oder exklusive der Detektion von Nies- und/oder Hustenattacken), was eine gezielte Rückmeldung an den Anwender, beispielsweise ähnlich einem KI-Trainer, ermöglicht. Dies kann zur Verbesserung der Atemtechnik und zur effektiveren Durchführung der Atemübungen beitragen. Ein weiterer Vorteil ist die Möglichkeit, detaillierte Informationen über den Atemvorgang zu erhalten, die für diagnostische und/oder therapeutische Zwecke genutzt werden können.

Zusammenfassend betrifft die vorliegende Erfindung ein Verfahren zur Bereitstellung und Optimierung von zumindest einer Information über die Wechselwirkung zwischen oraler und/oder trachealer und/oder nasaler Anwendung einer oder mehrerer Atemtherapie-/Atemtrainings-/ Entspannungs- /Schlaf- und Inhalations-/Medikationshilfen und/oder Medikationssysteme aller Art wie Dosieraerosolen mit und ohne Zählwerk, Mesh-/Ultraschall- oder Düsendruckluftvernebler oder Pulverinhalatoren mit der Atem-, Lungen-, Gehirn- und Bewegungsfunktion, Adhärenz, psychosozialer Faktoren und der häuslichen wie stationären Aufnahmen und Umgebungen eines Anwenders. Im Einzelnen kann die Erfindung zur Prävention, Diagnostik, Dokumentation, dem Monitoring und/oder einer daraus resultierenden Anleitung zur optimierten Inhalation, der Therapie und/oder dem Training der Atemzüge angewendet werden.

Bevorzugt kann in einem solchen Verfahren vorgesehen sein, dass ein Atemmuster zumindest eine Exhalation umfasst, vorzugsweise zusätzlich zumindest eine Inhalation und/oder wenigstens eine inhalatorische Pause und/oder eine exhalatorische Pause umfasst.

Allgemein kann das Verfahren ein Trainingsprogramm der korrekten Ausatmung umfassen, um die Funktion der Lunge im Rahmen einer Atemübung zu quantifizieren, aber auch einen Übungseffekt zu erzielen. Dies kann bis hin zu vollständigen Atemmustern, also den Verlauf von Einatmung, endinspiratorischer Pause, Ausatmung und endexspiratorischer Pause, sowie Wiederholungen des skizzierten Atemmusters pro Minute ergänzt werden. Durch eine mehrfache Durchführung und Erfassung über längere Zeiträume hinweg kann ein Monitoring erreicht und daraus tiefere Erkenntnisse über die Lungenfunktion des Anwenders abgeleitet werden.

Ferner kann vorgesehen sein, dass in dem Strömungskanal wenigstens eine Oszillationseinheit, vorzugsweise lösbar, derart aufgenommen ist, dass sie von der Luftströmung durch den Strömungskanal zum akustisch wahrnehmbaren Schwingen angeregt wird.

Bei der konkreten Ausgestaltung der Oszillationseinheit kann insbesondere vorgesehen sein, dass die wenigstens eine Oszillationseinheit entweder eine Stimmzungeneinheit mit wenigstens einer schwingfähig aufgehängten Stimmzunge, oder ein Schlauch mit zwei Zugängen ist, welcher in einer abgewinkelten oder gebogenen Rohrleitung derart gezwängt eingelegt ist, dass er zwischen seinen beiden Zugängen stets einen Knick ausbildet.

Während im Fall der Stimmzunge diese in ihrer Resonanzfrequenz in einem tiefen, angenehmen Ton zu schwingen beginnt, wenn ein Luftstrom aufgrund der Atmung des Anwenders durch den wenigstens einen Strömungskanal geleitet wird, drücken sich bei dem Schlauch Luftblasen durch den Schlauch hindurch und es ergibt sich eine andere Intensität und/oder Schwingfrequenzverlauf und/oder Dauer und/oder Amplitude. Hierüber kann die Auswerteeinheit erkennen, mit welchem Atemübungsgerät gearbeitet wird, so dass entsprechende Hinweise und Anleitungen gegeben werden können.

Ergänzend kann vorgesehen sein, dass die Oszillationseinheit auf eine Mundatmung und/oder auf eine Nasenatmung und/oder auf eine tracheale Atmung ausgelegt ist, insbesondere eine Stimmzungeneinheit mit wenigstens einer schwingfähig aufgehängten Stimmzunge und/oder einen Trichter und/oder einen in einen geknickten Verlauf gezwängten Schlauch und/oder ein Kugelventil und/oder ein Klappenventil aufweist.

So kann das Verfahren einer Wiederherstellung der funktionellen Atmung, also einer Umstellung gegebenenfalls von einer trachealen Atmung zur Mundatmung sowie letztlich hin zu einer Nasenatmung dienen. Die Nasenatmung ermöglicht es, die einströmende Luft bei der Einatmung zu filtern, entsprechend zu reinigen, zu befeuchten und zu erwärmen. Durch die Degeneration der Atemwege sowie durch akute und/oder chronische Atemwegserkrankungen können Anwender oft nur über den Mund atmen. Bei einem Luftröhrenschnitt oder nach Bauchoperationen ist die Atmung sogar nur über eine Trachealkanüle möglich. Diese Atmung ist oft flach und nicht in den Bauch hinein, sondern meist in die Brust. Eine solche Atmung führt häufig zu Hyperventilation, also sehr kurzen, ruckartigen Atemzügen, die vor allem von einer schnellen Einatemphase gefolgt von einer verkürzten Ausatemphase geprägt sind. Diese Atemweise steht allerdings nicht nur für chronisch kranke Anwender, sondern allgemein für gestresste und sehr angespannte Menschen. Durch diese dysfunktionale Atmung kann die Infekthäufigkeit bis hin zu chronischen Blockaden der oberen Atemwege steigen. Zudem können Wucherungen in den oberen Atemwegen, sogenannte Polypenbildungen oder auch Septumdeviationen und ähnliches vorliegen, die eine natürliche Atmung hemmen oder unmöglich machen. Auch chronische Blockaden der Atemwege können vorhanden sein. Alle haben eines gemein, die Betroffenen, ca. 40% der Weltbevölkerung atmen chronisch nur über den Mund. Sie setzen sich damit unnötig zusätzlichen Risiken sowie Infekten aus.

Das vorliegende System und Verfahren soll hier nun auf einfache Weise Abhilfe schaffen. Durch die Ausgestaltung des wechselbaren Atemaufsatzes kann der Anwender sich zum einen an das Training und/oder die Therapie über die Trachealkanüle oder den Mund gewöhnen. Das System und das Verfahren können durch die Tonalität - es ist eine Sonifikation der Atmung, des Atemzyklus und der Atemtechnik, also der Atemzüge, vorgesehen, dessen Tiefe und Regelmäßigkeit sowie die tägliche Adhärenz im Abgleich mit dem Wohlbefinden des Anwenders aufgenommen, analysiert und ihm, bei Bestätigung, weitere Schritte hin zur Anwendung der oralen und/oder nasalen Therapie zur Auflösung der dysfunktionalen Atmung vorgeschlagen. Fühlt er sich wohl und hat er ein gewisses Level an Art und Ausgestaltung seiner Atemzüge erreicht, kann die Therapie über die Mund- bzw. Nasenapplikation begonnen werden.

Bei der nasalen Anwendung lässt sich feststellen, inwiefern überhaupt Blockaden der oberen Atemwege wie eine ausgeprägte Wechselatmung, akute, sich anbahnende Erkältungen durch Niesen und/oder Husten, Septumdeviationen oder Polypen vorliegen. Besteht für den Anwender nur auf einem Nasenloch die Möglichkeit, ein akustisch wahrnehmbares Signal zu erzeugen und auf dem anderen Nasenloch nicht, spricht dies für eine Komplikation und/oder für die sogenannte "Ruhephase" der wechselseitigen Nasenatmung, in der die Atmung nur eines Nasenlochs "leicht blockiert ist". Damit das System dies erkennen kann, wird es darauf programmiert, dass unterschiedliche Frequenzen und/oder Luftströmungen mit oder ohne Kombination von Soundmustern für das Niesen und Husten klar erkennbar sind.

Für die Nase kann das System mit einem anderen Luftstrom ausgestattet werden. Dieser Luftstrom wird vorzugsweise zum einen nicht mit Luftlöchern, sondern mit einem mit Sekret nicht verlegbaren Kanal ausgestattet. Zudem erfolgt über diesen anderen Kanal ein differenzierbares akustisches Signal. Durch die duale Ausgestaltung kann immer zwischen Atemzügen bei der Ein- und Ausatmung differenziert werden. Erklingt dann kein Ton, ist von einer Blockade auszugehen. Diese Blockaden können allerdings auch lediglich in Sekretretentionen bestehen. Um dies abzuklären, kann das System zunächst eine Sekretolyse mit Inhalation vorschlagen. Kommt es nicht zum gewünschten Effekt, so kann die Applikation von abschwellenden Nasentropfen vorgeschlagen werden. Kommt es hierbei nur zur Eröffnung eines Nasenlochs, wird es sich um eine einseitige Polypenbildung oder Septumdeviation handeln. Dies ist dann mittels der Gabe von ätherischen Ölen lösbar oder führt zu einer direkten Empfehlung eines HNO-Besuchs mit Terminvergabe, da dann weitere Schritte eingeleitet werden müssen.

Damit die Wechselatmung der Nase und/oder eine gegebenenfalls primär bestehende Nasenblockade von einem System erkannt werden kann, ist es möglich, dass das Nasenstück der Erfindung in folgender Art auszugestalten.

Bevorzugtermaßen kann die Auswerteeinheit ein inspiratorisches Strömungsgeräusch von einem exspiratorischen Strömungsgeräusch je Nasenloch oder bei der oralen bzw. trachealen Atmung unterscheiden und die beiden Strömungsgeräusche für den Vergleich mit dem Atemmuster separat auswerten.

In diesem Kontext bedeutet die Unterscheidung der Parameter, dass die Auswerteeinheit in der Lage ist, die spezifischen Werte der Strömungsgeräusche während der Einatmung und der Ausatmung getrennt zu analysieren. Es ist insbesondere möglich, dass eine Stimmzunge verwendet wird, die in beiden Strömungsrichtungen anspricht, oder dass für jede Strömungsrichtung eine eigene Stimmzunge vorgesehen ist, die in einer eigenen Tonhöhe schwingt, um diese leicht unterscheiden zu können. Auch für den Anwender wird so eine akustische Rückmeldung gegeben.

Es kann eine unterschiedliche Ausgestaltung von genauen Erkennungsmerkmalen wie Frequenzgang, Amplituden, Oszillationen gemäß Druck- und Fluss sowie der entsprechenden Dezibel für die Bestimmung des Druck- und Flussprofils bei einem genauen Abstand zum mobilen Endgerät sowohl bei der Ein- und Ausatmung als auch bei der jeweils folgenden endinspiratorischen und endexspiratorischen Pause, bei der kein akustisches Signal erfolgt, aber es durch die Abfolge der inspiratorischen und exspiratorischen Tonalität nachvollziehbar ist, für die Bestimmung eines Atemzugs über Mund und/oder Nase von verschiedenen Atemtherapie- und Atemtrainingsgeräten zu deren genauer Bestimmung ermittelt werden.

Unterschiedliche Strömungsausprägungen für die nasale, orale oder tracheale Anwendung können akustisch unterschieden werden. Je nach Funktion eingesetzter Ventile - etwa Kugelventil, Trichterventil und/oder Klappenventil sowie Schläuche und/oder Stimmplattenventile - ergeben sich unterschiedliche Frequenzgänze. Auch verschiedene Ummantelungen, die entsprechend charakteristische Frequenzgänge, Druck- und Flusskurven sowie Amplituden und Lautstärken bewirken, können von der Auswerteeinheit ebenso selbsttätig erkannt werden wie einfache Ventil- oder Stenosengeräusche.

Insoweit kann ergänzend vorgesehen sein, dass dem Strömungskanal eine veränderliche Stenose zugeordnet ist, deren Öffnungsgrad anhand eines Störgeräuschs und/oder einer Veränderung des Frequenzverlaufs durch die Auswerteeinheit ermittelt wird. Ebenfalls kann mit anderen variablen Einstellungen des Atemübungsgeräts gearbeitet werden, etwa dem Abstand eines Trichters und/oder eines Klappenventils und/oder eines kombinierten Schlauchventils. Soweit im Weiteren von einer Stenose gesprochen wird, gelten deren Einflussmöglichkeiten auch hierfür.

Dies bedeutet, dass die Stenose, die innerhalb des Gehäuses des Atemübungsgeräts den wenigstens einen Strömungskanal verengt, so angepasst werden kann, dass ihre Öffnungsgröße variabel ist. Diese Anpassungsfähigkeit ermöglicht es, den Widerstand, den die Luftströmung in dem wenigstens einen Strömungskanal erfährt, zu modifizieren, was für unterschiedliche therapeutische Anforderungen und individuelle Anwenderbedürfnisse von Vorteil ist. Der Öffnungsgrad der Stenose kann dabei anhand eines Störgeräuschs durch die Auswerteeinheit erfasst werden, denn je enger die Stenose, desto mehr Verwirbelungen treten im Bereich des Luftaustritts auf. Dies bedeutet, dass die Auswerteeinheit in der Lage ist, akustische Signale, die durch die Luftströmung und die Interaktion mit der Stenose erzeugt werden, zu analysieren und daraus Rückschlüsse auf die aktuelle Öffnungsgröße der Stenose zu ziehen. Diese akustische Erfassung bietet eine präzise und kontinuierliche Überwachung der Stenosenstellung bzw. der verschiedenen Ventileinstellungen, unabhängig davon ob es ein Klappen-, Trichter-, Kugel- oder sonstiges Ventil ist, ohne dass mechanische Sensoren erforderlich sind, was die Komplexität und die potenzielle Fehleranfälligkeit des Systems reduziert. Der Frequenzgang bzw. das Strömungsgeräusch wird entsprechend beeinflusst.

Ergänzend kann es sinnvoll sein, wenn die Auswerteeinheit das Strömungsgeräusch bei einer Atmung durch ein mit dem Atemübungsgerät, vorzugsweise lösbar, verbundenes Nasenstück, Mundstück oder eine Trachealkanüle anhand von Intensität und/oder Frequenzverlauf und/oder Dauer und/oder Amplitude des erfassten Strömungsgeräuschs unterscheidet. Grundsätzlich ist vorgesehen, dass diese verschiedenen Bedienungsformen des Atemübungsgeräts alternativ eingesetzt werden können. Auch eine Detektion bzw. Integration der Nies- und/oder Hustengeräusche an sich sind denkbar.

In konkreter Weiterbildung kann vorgesehen sein, dass die Auswerteeinheit einen Ablauf mehrerer Atemzyklen vorgibt und ermittelt, wie viele dieser Atemzyklen innerhalb einer Atemübung ausgeführt werden, wobei vorzugsweise ein Trainingserfolg über einen längeren Zeitraum hinweg erfasst und die Trainingsintensität und -dauer von der Auswerteeinheit an den Fortschritt angepasst wird. Diese gespeicherten Atemübungen bieten dem Anwender eine strukturierte Anleitung zur Durchführung der Atemübungen, indem sie über die Ausgabemittel des Datenverarbeitungsgeräts bereitgestellt werden. Dies führt zu einer höheren Motivation und einem besseren Therapieerfolg, da der Anwender durch die unterschiedlichen Übungen und die direkte Rückmeldung kontinuierlich gefordert und gefördert wird. Die Integration der Ausgabemittel zur Bereitstellung der Anweisungen stellt sicher, dass der Anwender die Übungen korrekt durchführt und die gewünschten therapeutischen Effekte erzielt werden.

Zudem kann vorgesehen sein, dass die Auswerteeinheit eingangs einer Atemübung einen Initialschritt vornimmt, bei dem Intensität und/oder Schwingfrequenzverlauf und/oder Dauer und/oder Amplitude der Strömungsgeräusche des Atemübungsgeräts sowie Umgebungsgeräusche erfasst werden, wobei während der nachfolgenden Atemübung ein Bereich um die Intensität und/oder Schwingfrequenzverlauf und/oder Dauer und/oder Amplitude des Strömungsgeräuschs des Atemwegstherapiegeräts mithilfe eines Bandfilters isoliert und Umgebungsgeräusche ausgefiltert werden. Diese Erfassung ist entscheidend, um eine genaue Basislinie für die nachfolgende Analyse zu etablieren. Während der Atemübung wird ein Bereich um die Tonhöhe des Strömungsgeräusches mithilfe eines Bandfilters isoliert, wodurch Umgebungsgeräusche ausgefiltert werden. Dies ermöglicht eine klare und ungestörte Analyse des vom Anwender durch dessen Atmung erzeugten Strömungsgeräusches. Der Einsatz eines Bandfilters zur Isolierung der relevanten Tonhöhe stellt sicher, dass die Auswerteeinheit präzise Daten über Intensität und/oder Schwingfrequenzverlauf und/oder Dauer und/oder Amplitude des Strömungsgeräusches während der Inspirations- und Exspirationsvorgänge des Anwenders erfasst und möglichst wenig Störgeräusche behandeln muss. Durch die Filterung der Umgebungsgeräusche wird die Wahrscheinlichkeit von Fehlinterpretationen oder Störungen minimiert, was zu einer zuverlässigeren und effektiveren Atemtherapie führt.

Insbesondere kann hierbei vorgesehen sein, dass die Auswerteeinheit im Rahmen des Initialschritts empfangene Tonsignale auf das Klangbild eines Ultraschallverneblers oder anderer Medikamentenvernebler prüft. Strömungs- oder auch Auslösegeräusche der Medikamentencontainer können erkannt und aufgezeichnet sowie analysiert werden. Auch der Flow bei der Einatmung über Medikamentencontainer kann über die Amplitude, Frequenz, Periode und Intensität genau bestimmt, abgetragen und gegebenenfalls vorab trainiert oder korrigiert werden. So kann die Medikamentenverneblung jedweder Art mit und ohne Kombination mit der Atemtherapie genutzt werden, um die Wechselwirkung von Infekten, Krankenhausaufenthalten und dem Bedarf der Medikation in Kombination mit den oben genannten Hilfsmitteln wie dem Atemtraining und der Atemtherapie sowie Entspannungs-, Inhalierhilfen und Schlafhilfen genauer zu überwachen und gegebenenfalls deren Kombination optimieren zu können.

Zudem kann die Auswerteeinheit dem Atemübungsgerät entweder baueinheitlich zugeordnet sein, oder einem Datenverarbeitungsgerät zugeordnet sein, wobei dem Atemübungsgerät elektronische Kommunikationsmittel zum Datenaustausch mit dem Datenverarbeitungsgerät zugeordnet sind, wobei zumindest Daten zur Erkennung des Typs des Atemübungsgeräts über die elektronischen Kommunikationsmittel durch das Atemübungsgerät an das Datenverarbeitungsgerät übermittelbar sind.

Kombiniert mit Wearables und/oder Schlafsystemen und/oder Beatmungssystemen kann das erfindungsgemäße System zusätzliche Daten in die Bewertung einfließen lassen. Allem voran stehen hierbei die Herzfrequenz, die Sauerstoffsättigung und die Blutdruckdaten. Durch die Absenkung der Atemfrequenz kommt es zu einer Absenkung der Herzfrequenz, sowie je nach Ausgestaltung des Atemzyklus' auch zu einer Änderung des Gasaustausches. Diese hat wiederum Einfluss auf den Blutdruck. Entsprechend können durch regelmäßiges Training und/oder Therapie diese Parameter optimal beeinflusst werden. Durch die Reduktion der Atemfrequenz kommt es zudem zu einer Beruhigung des Anwenders. Dieser kann dadurch, eine regelmäßige Anwendung vorausgesetzt, einfacher einschlafen.

In Weiterbildung dieses Falles kann vorgesehen sein, dass auf dem Datenverarbeitungsgerät Atemübungen mit unterschiedlichen Abläufen, insbesondere hinsichtlich Intensität und/oder Schwingfrequenzverlauf und/oder Dauer und/oder Amplitude der Strömungsgeräusche, Einstellungen und/oder Tonfolgen, gespeichert sind und über die Ausgabemittel der Auswerteeinheit Anweisungen zur Durchführung dieser Atemübungen, vorzugsweise einschließlich einer Einbettung in Musik- und Videodaten zur Begleitung der Atemübungen, bereitgestellt werden. Dies kann den Anwender dabei unterstützen, den Atemrhythmus individuell für Entspannung, Schlaf oder Meditation zusätzlich anzupassen.

Es kann weiter vorgesehen sein, dass ein Tages- und/oder Wochenplan, oder auch längerfristige Therapiepläne, in das Datenverarbeitungsgerät eingebunden werden. Dieser Plan ermöglicht es, dass das Datenverarbeitungsgerät über seine Ausgabemittel zu vorgegebenen Zeiten Erinnerungen an den Anwender ausgibt. Die Integration eines Tages- und/oder Wochenplans in das Datenverarbeitungsgerät bringt mehrere Vorteile mit sich. Erstens unterstützt es die regelmäßige Durchführung der Atemübungen, indem es den Anwender zu festgelegten Zeiten an die Übungen erinnert. Dies fördert die Einhaltung des Therapieplans und kann zu besseren Therapieergebnissen führen. Zweitens kann der Plan individuell auf die Bedürfnisse und den Tagesablauf des Anwenders abgestimmt werden, was die Flexibilität und Benutzerfreundlichkeit des Verfahrens erhöht. Schließlich kann die Integration eines solchen Plans auch die Motivation des Anwenders steigern, da er durch die Erinnerungen kontinuierlich an die Wichtigkeit und den Nutzen der Atemübungen erinnert wird.

Bevorzugtermaßen können zudem dem Datenverarbeitungsgerät Mittel zur Dateneingabe und/oder zum Erfassen und/oder zum Abrufen von Daten von einer Datenbank und/oder zum selbsttätigen Vorschlagen oder Festlegen von Daten zugeordnet sein, wobei die Daten
- Anwendergruppierungen und/oder Vorerkrankungen eines Anwenders, insbesondere Obstruktionsgrade der Bronchien und/oder Häufigkeiten von Exazerbationen, Komorbiditäten und/oder Symptomlasten,
- und/oder Parameter von stationärer und ambulanter Rehabilitationsmaßnahmen und/oder stationärer und ambulanter therapeutischer und/oder ärztlicher Betreuung oder einer Medikamentierung eines Anwenders,
- und/oder Orts- und/oder Kalenderdaten, Veranstaltungsdaten oder Gesundheitswarnungen öffentlicher Stellen,
- und/oder Positionsdaten, sowie vorzugsweise auch anonymisierte Anwenderdaten anderer Anwender
umfassen.

Besonders relevant sind hierbei Daten zu Obstruktionsgraden der Bronchien, Häufigkeiten von Exazerbationen, Komorbiditäten und Symptomlasten. Die Integration der Mittel zur Dateneingabe und -erfassung bietet mehrere Vorteile. Zum einen ermöglicht sie eine präzisere und individuellere Anpassung der Atemübungen an die spezifischen Bedürfnisse und Gesundheitszustände der Anwenderen. Dies ist besonders wichtig für Anwender mit chronischen Atemwegserkrankungen, da die Therapie so auf die jeweiligen Obstruktionsgrade der Bronchien und die Häufigkeit von Exazerbationen abgestimmt werden kann. Die erfassten Daten können zudem genutzt werden, um Muster und Trends zu identifizieren, die auf eine Verschlechterung des Gesundheitszustands hinweisen, sodass frühzeitig Gegenmaßnahmen ergriffen werden können.

Besonders vorteilhaft wird es empfunden, wenn die Daten Anwendergruppierungen und/oder Vorerkrankungen des Anwenders, insbesondere Obstruktionsgrade der Bronchien und/oder Häufigkeiten von Exazerbationen, Komorbiditäten und/oder Symptomlasten, Parameter von Krankenhausaufenthalten oder Medikamentierung, Orts- und/oder Kalenderdaten, Veranstaltungsdaten oder Gesundheitswarnungen öffentlicher Stellen, und/oder Positionsdaten, sowie vorzugsweise auch anonymisierte Anwenderdaten anderer Anwender umfassen. In einem solchen Fall kann der Anwender verfahrensgemäß angeleitet werden, wann, wie (nasal/oral/tracheal) und wo ein Atemtraining und/oder eine Atemtherapie mit und ohne Inhalation bzw. Medikationseinnahme vermehrt durchgeführt werden sollte, mit oszillierenden Atemtherapie- und - trainingsgeräten mit und ohne Feuchtinhalation bzw. andere Medikationsgaben durch Meshvernebler, Dosieraerosole und/oder auch Pulverinhalatoren, etc. Dies kann in einem solchen Fall basierend auf der Wechselwirkung von Ortsdaten, Kalenderdaten und Daten von Gesundheitsbehörden und -institutionen erfolgen. Hierbei kommt es beispielsweise darauf an, ob ein verstärkter Aufenthalt in geschlossenen Räumen stattfindet, ob eine verstärkte Aktivität und Kontakt mit vielen Leuten in einem Restaurant oder beim Einkaufen festgestellt wird, ob es beispielsweise Karnevals- oder Weihnachtszeit ist und mit verstärkter Versammlungstätigkeit zu rechnen ist. Hierbei ist auch die Ortsangabe wichtig, um auf lokale Bräuche und Jahreszeiten Rücksicht nehmen zu können. Hinsichtlich der Gesundheitsdaten kann bei bekannten Grippewellen und anderen Daten zur Verbreitung und damit bei einer verstärkten Expositionsmöglichkeit gegenüber Viren und Bakterien reagiert und zusätzliche Vorsicht und vorbeugende therapeutische Maßnahmen angemahnt werden.

Darüber hinaus ist die Verknüpfung von Ortsdaten für den Anfang der Heizperiode relevant. Dann halten sich Menschen mehr in geschlossenen Räumen auf. Das Infektrisiko steigt u.a. auch durch den Kontakt mit Kleinkindern. Über geteilte Positionsinformationen, Ortungs-, Kalender- und/oder Eventdaten aus dem Internet können Infektherde vermieden werden. Es können entsprechende Hinweise aus den Daten abgeleitet werden, die durch Auswertungsdaten immer wieder angepasst und verfeinert werden. Der Anwender kann von der Auswerteeinheit aufgefordert werden, Befeuchtungsmaßnahmen, insbesondere regelmäßiges Stoßlüften bzw. alternativ Einnahme von befeuchtenden Nasensprays, Feuchtinhalation, Einnahme von z.B. Kortikosteroiden durchzuführen. Aber auch bei der trachealen Anwendung kann eine gewisse Routine mit dem Ableich von Krankenhausdaten erfolgen und eine entsprechende Anwendungshäufigkeit vorgeschlagen werden. Auch für eine verbesserte Schlafhygiene, insbesondere eine Nasenatmung, kann vermehrt diese vor dem Schlafen nochmals angewendet und dann der Anwender durch ein Atemmuster der Meditation bzw. abgeschwächt der Entspannung, zu einer besseren Schlafhygiene, allerdings hier vorzugsweise rein über eine akustische und keine visuelle Anleitung, angeleitet werden. Ein Abgleich mit der Länge und Intensität des Schlafes und entsprechendem Vorschlag für die nächste Nacht sind hier als vorteilhaft zu nennen, da gerade die körperliche Regeneration im Schlaf besonders gefördert wird.

Hierbei ist es besonders vorteilhaft, wenn dem Datenverarbeitungsgerät eine künstliche Intelligenz zugeordnet ist, welche eingegebene und/oder erfasste Daten und/oder Parameter auswertet und Vorschläge für Atemübungen oder andere Daten und/oder Parameter unterbreitet. Diese Künstliche Intelligenz wertet eingegebene und/oder erfasste Daten aus und unterbreitet Vorschläge für weitere Atemübungen. Die Integration der Künstlichen Intelligenz in das Datenverarbeitungsgerät ermöglicht eine erweiterte Analyse der erfassten Daten. Sie kann Muster und Trends in den Atemdaten erkennen und darauf basierend personalisierte Vorschläge für weitere Atemübungen unterbreiten. Dies bietet dem Anwender eine maßgeschneiderte Therapie, die auf seinen individuellen Fortschritten und Bedürfnissen basiert. Ein Vorteil dieser Ausführungsform ist die Möglichkeit, die Effizienz und Wirksamkeit der Atemübungen zu steigern, indem die Künstliche Intelligenz kontinuierlich lernt und sich an die spezifischen Anforderungen des Anwenders anpasst. Ein weiterer Vorteil ist die Verbesserung der Anwenderenmotivation und -bindung durch die Bereitstellung von personalisierten Übungsvorschlägen und Rückmeldungen in Echtzeit. Die Künstliche Intelligenz trägt dazu bei, die Therapieergebnisse zu optimieren und die langfristige Gesundheit und das Wohlbefinden des Anwenders zu fördern. Eine individualisierte Betreuung ist hierdurch auch mit weniger Personal realisierbar.

In vorteilhafter Ausgestaltung kann zudem vorgesehen sein, dass die Rückmeldung ein Punktesystem, eine Fortschrittsvisualisierung oder zumindest ein Gamification-Element umfasst. Dies bietet dem Anwender eine motivierende und interaktive Benutzererfahrung. Das Punktesystem ermöglicht es dem Anwender, für korrekt durchgeführte Atemübungen Punkte zu sammeln, was zu einer gamifizierten und belohnenden Erfahrung führt. Dies kann die Motivation und das Engagement des Anwenders erhöhen, da er durch das Sammeln von Punkten Fortschritte sichtbar machen kann. Eine Fortschrittsvisualisierung bietet dem Anwender eine grafische Darstellung seines Fortschritts über die Zeit. Dies kann in Form von Diagrammen, Balken oder anderen visuellen Indikatoren erfolgen, die dem Anwender eine klare und verständliche Rückmeldung über seine Leistung und Verbesserungen geben. Ein Gamification-Element kann verschiedene spielerische Elemente umfassen, wie z.B. das Erreichen von Levels, das Freischalten von Belohnungen oder das Erreichen von Meilensteinen. Diese spielerischen Elemente können das Training unterhaltsamer und ansprechender gestalten, was wiederum die Compliance und die langfristige Nutzung des Geräts fördern kann. Darüber hinaus kann die Verwendung solcher interaktiven und motivierenden Elemente dazu beitragen, die Therapieergebnisse zu verbessern, indem sie den Anwender dazu anregen, sich stärker in den Therapieprozess einzubringen.

Weiter kann vorgesehen sein, dass das Datenverarbeitungsgerät ein Laptop, ein Tablet, ein Smartphone, ein Wearable, ein Lungenfunktionsgerät, ein CPAP, ein Cough-Assist und/oder ein Hustendetektor ist.

Schließlich erscheint es sinnvoll, wenn die Ausgabemittel einen Bildschirm und/oder einen Lautsprecher, etwa einen Kopfhörer, umfassen. Hierdurch können sowohl visuelle als auch akustische, sowie darüberhinaus haptische Signale genutzt werden, um mit dem Anwender zu kommunizieren.

Die vorstehend beschriebene Erfindung wird im Folgenden anhand eines Ausführungsbeispiels näher erläutert.

Es zeigen
- Figur 1: eine Ausführungsform eines Atemübungsgeräts und eines Datenverarbeitungsgeräts, die zur Durchführung von Atemübungen über ein Mundstück verwendet werden in einer schematischen Schnittdarstellung,
- Figur 2: eine Ausführungsform eines Atemübungsgeräts mit einem Nasalstück, wobei das Atemübungsgerät mit einem Datenverarbeitungsgerät verbunden ist,
- Figur 3: eine Ausführungsform eines Atemübungsgeräts mit einem Gehäuse und einer drehverstellbaren Stenose, sowie
- Figur 4: eine weitere Ausführungsform eines Atemübungsgeräts mit einer Oszillationseinheit in Form eines Schlauchs.

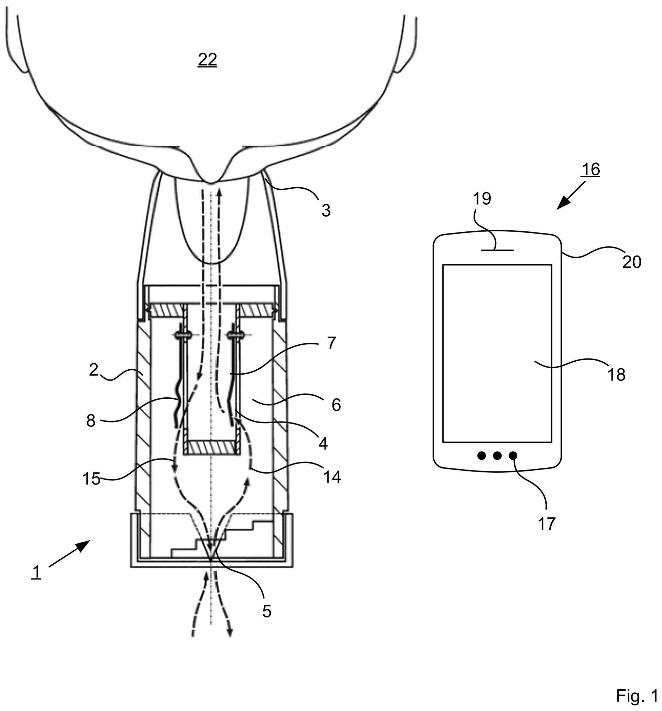

Figur 1 zeigt eine schematische Darstellung eines Systems zur Durchführung von Atemübungen, bestehend aus einem Atemübungsgerät 1 und einem Datenverarbeitungsgerät 16. Das Atemübungsgerät 1 umfasst ein Gehäuse 2, das einen Strömungskanal 6 zwischen einem Mundstück 3 und einer Stenose 5 ausbildet. Innerhalb des Strömungskanals 6 sind zwei schwingfähig aufgehängte Stimmzungen 7 und 8 angeordnet, die durch eine Luftströmung in Schwingung versetzt werden können. Eine erste Stimmzunge 7 ist für den Inspirationsvorgang 14 und eine zweite Stimmzunge 8 für den Exspirationsvorgang 15 vorgesehen, so dass die Stimmzungen 7 und 8 in unterschiedlichen Tonhöhen betrieben werden können. Für den Anwender 22 ergibt sich dadurch eine charakteristische Tonfolge.

Das Datenverarbeitungsgerät 16 ist ein herkömmliches Smartphone mit einem Programmprodukt, welches das vorliegende Verfahren durchführt. Ausgestattet ist es mit einem Mikrofon 17, einer Auswerteeinheit 20, wie etwa dem Programm und den dafür erforderlichen Prozessoren, Registern und Speichern, sowie mit Ausgabemitteln in Form eines Bildschirms 18 und eines Lautsprechers 19. Das Smartphone mit dem Mikrofon 17 wird im Rahmen einer Atemübung so positioniert, dass es die Schwingungen der Stimmzungen 7 und 8 erfasst, die durch die Atemluft des Anwenders 22 erzeugt werden. Die erfassten Daten werden innerhalb des Datenverarbeitungsgeräts 16 an die Auswerteeinheit 20 weitergeleitet, welche die Schwingfrequenz und Intensität der Schwingungen während der Inspirationsvorgänge 14 und Exspirationsvorgänge 15, sowie die Dauer der dazwischen liegenden inspiratorischen und exspiratorischen Pausen analysiert und aufzeichnet. Hieraus ermittelt die Auswerteeinheit 20 ein Atemmuster, welches mit Sollvorgaben verglichen und unter Berücksichtigung der weiteren in das Datenverarbeitungsgerät 16 eingegebenen Daten des Anwenders 22, sowie darüber hinaus unter Verwendung zusätzlicher Daten aus Datenbanken, z.B. für Veranstaltungen, ausgewertet wird. Es kann hierdurch berücksichtigt werden, ob sich der Anwender 22 an dem Ort und zu der Zeit einer Veranstaltung mit vielen Menschen befindet, so dass in diesem Fall aufgrund von Infektionsgefahren andere Maßnahmen empfohlen werden können als an anderen Orten oder zu anderen Zeiten.

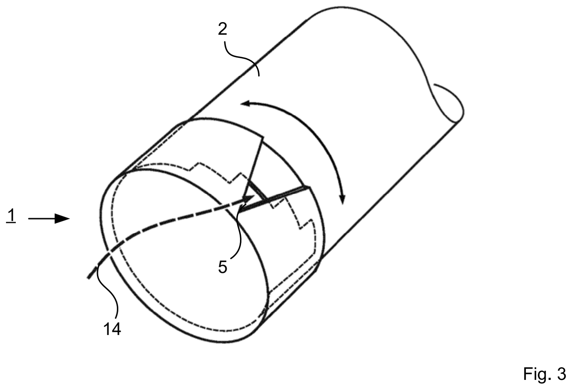

Die Stenose 5 im Strömungskanal 6 ist in ihrer Öffnungsgröße veränderlich, was es ermöglicht, den Widerstand der Luftströmung anzupassen. Dies ist im Einzelnen in Figur 3 gezeigt. Die Auswerteeinheit 20 kann anhand eines über Luftverwirbelungen erzeugten Störgeräuschs an der Stenose 5 den Öffnungsgrad der Stenose 5 erfassen und dem Anwender 22 über die Ausgabemittel 18 und 19 Rückmeldungen geben, die eine Anpassung der Stenose 5 anregen, wenn sich dies aus der Auswertung der Atemübungen ergibt. Soweit die Stenose 5 elektronisch verstellbar ist, kann eine solche Anpassung auch direkt auf Veranlassung der Auswerteeinheit 20 über einen hier nicht gezeigten Stellmotor vorgenommen werden.

Die Auswerteeinheit 20 unterscheidet die Schwingfrequenzen der Schwingungen während des Inspirationsvorgangs 14 und des Exspirationsvorgangs 15, um ein Atemmuster des Anwenders 21 zu bemessen. Zusätzlich werden die Dauer der Schwingungen sowie die Ruhephasen zwischen den Atemvorgängen erfasst und ausgewertet, um die Regelmäßigkeit der Atemzyklen zu bestimmen.

Zu Beginn einer Atemübung führt die Auswerteeinheit 20 einen Initialschritt durch, bei dem die Tonhöhe der Stimmzungen 7 und 8 sowie Umgebungsgeräusche erfasst werden. Ein Bandfilter isoliert während der Atemübung einen Bereich um die Tonhöhe der Stimmzungen 7 und 8 herum, um Umgebungsgeräusche auszublenden. Hier ist das Atemübungsgerät 1 mit einem für die Mundatmung ausgelegten Mundstück 3 versehen, dem Stimmzungen 7 und 8 in einer Stimmzungeneinheit 4 zugeordnet sind, die zur Mundatmung passen, insbesondere einen stärkeren Luftstrom erfordern um zum Schwingen angeregt zu werden. Die Stimmzungeneinheit 4 kann als Ganzes mit dem Mundstück 3 verbunden sein, so dass ein Wechsel auf einen anderen Atemaufsatz, wie etwa ein Nasenstück 21, auch zu einer anderen Bestückung mit Stimmzungen 7 und 8 führt. Alternativ kann die Stimmzungeneinheit 4 auch separat entnommen und nach Bedarf ausgetauscht werden.

Das Atemübungsgerät 1 kann in einer alternativen Ausgestaltung, die hier nicht gezeigt ist, auch mit elektronischen Kommunikationsmitteln ausgestattet sein, die einen Datenaustausch mit dem Datenverarbeitungsgerät 16 ermöglichen. Dies umfasst dann insbesondere Daten zur Erkennung des Typs des Atemübungsgeräts 1 und die Tonhöhen der Stimmzungen 7 und 8. Eine elektronische Erfassung und Übermittlung der Stenoseeinstellungen 5 an das Datenverarbeitungsgerät 16 ist dann ebenfalls möglich. Auch die vollständige Integration der Auswerteeinheit in das Atemübungsgerät kann in diesem Zusammenhang erfolgen.

Auf dem Datenverarbeitungsgerät 16 sind verschiedene Atemübungen mit unterschiedlichen Abläufen gespeichert. Diese Übungen können hinsichtlich Dauer der Inspirationsvorgänge 14, Dauer der Exspirationsvorgänge 15, der dazwischenliegenden Pausen, der Anzahl der Wiederholungen, der vorgegebenen Schwingfrequenz, Einstellungen und/oder Tonfolgen variieren und dem Anwender 22 über die Ausgabemittel 18 und 19 Anweisungen zur Durchführung bereitstellen. Während der Atemübungen führt das Datenverarbeitungsgerät 16 eine Adhärenzmessung durch, bei der die gemessenen Tonhöhen der Stimmzungen 7 und 8, auch über die Zeit, mit den Vorgaben der Atemübungen verglichen werden.

Das Datenverarbeitungsgerät 16 kann auch einen Tages- und/oder Wochenplan enthalten, der den Anwender 22 zu vorgegebenen Zeiten an die Durchführung der Atemübungen erinnert. Zudem sind Mittel zur Dateneingabe und/oder zum Erfassen oder auch Abrufen von Daten aus Datenbanken vorhanden, die Anwendergruppierungen und/oder Vorerkrankungen sowie gegebenenfalls weitere Daten umfassen.

Eine künstliche Intelligenz im Datenverarbeitungsgerät 16 kann die eingegebenen, erfassten und abgerufenen Daten auswerten und Vorschläge für weitere Atemübungen unterbreiten. Das System kann zudem ein Punktesystem, eine Fortschrittsvisualisierung oder Gamification-Elemente umfassen, um dem Anwender 22 Rückmeldungen über Trainings- und Therapieerfolge zu geben.

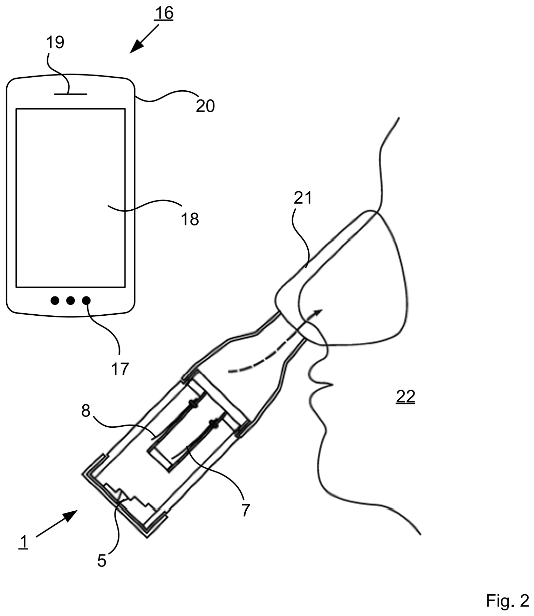

**In** einer alternativen Ausführungsform gemäß Figur 2 kann der Atemaufsatz als Nasenstück 21 ausgebildet sein. Das Datenverarbeitungsgerät 16 kann in Form eines Smartphones oder eines Wearables gestaltet oder vollständig in das Atemübungsgerät 1 integriert sein.

Figur 3 zeigt eine perspektivische Ansicht des unteren Endes eines Atemübungsgeräts 1. Das Gehäuse 2 des Geräts bildet einen Strömungskanal aus, der zwischen einer Atemöffnung und einer Stenose 5 verläuft. Die Stenose 5 ist innerhalb des Strömungskanals 6 positioniert und kann in ihrer Öffnungsgröße verändert werden, um den Luftstrom, hier einen Inspirationsvorgang 14, zu regulieren.

Das Gehäuse 2 ist so gestaltet, dass es eine Luftströmung durch den Strömungskanal 6 ermöglicht, wenn ein Anwender 22 durch eine Atemöffnung mit einem Mundstück 3 oder einem Nasenstück 21, oder auch durch eine Trachealkanüle atmet. Die Stenose 5 ist so angeordnet, dass sie eine Verengung des Strömungskanals 6 bewirkt, wodurch die Geschwindigkeit der Luftströmung erhöht wird und somit eine Schwingung der im Strömungskanal 6 befindlichen Stimmzungen 7 bzw. 8 angeregt wird.

Die Stenose 5 kann mechanisch oder elektronisch angepasst werden, um den Öffnungsgrad zu verändern. Vorliegend besteht eine Änderungsmöglichkeit im gegenseitigen Verdrehen eines unteren geschlitzten Elements gegen ein oberes, gestuftes Element. Durch eine Drehung des geschlitzten Teils nach rechts wird die Stenose 5 weiter geöffnet, durch eine Drehung des geschlitzten Teils nach links weiter geschlossen. Diese Anpassung ermöglicht eine Feinabstimmung des Atemwiderstands, was für die Durchführung spezifischer Atemübungen wichtig ist. Die Veränderung der Öffnungsgröße der Stenose 5 kann neben einer Drehbewegung auch durch eine andere geeignete Mechanik erfolgen.

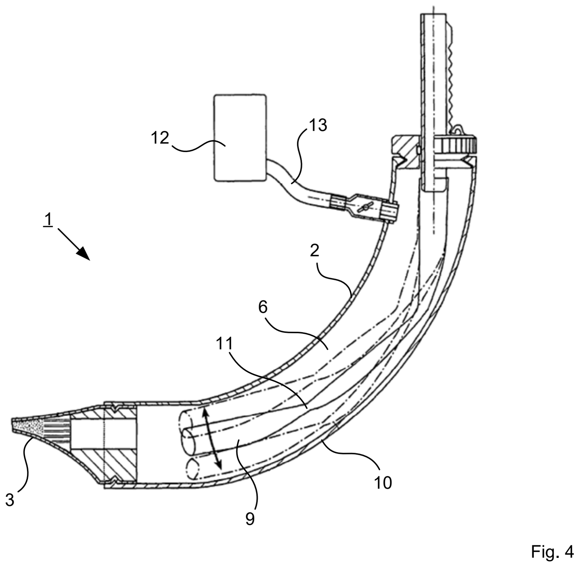

Figur 4 zeigt schließlich eine weitere Ausgestaltung eines Atemübungsgeräts 1, welches anstelle der bisher gezeigten Stimmzungen mit einem Schlauch 9 arbeitet. Dieser liegt gezwängt in einer gebogenen Rohrleitung 10, so dass der Schlauch 9 in der Rohrleitung 10 stets mindestens einen Knick 11 aufweist. Bei der Inspiration durch das Mundstück 3 kann ein Anwender Luft durch den Schlauch 9 ansaugen, wodurch sich der Knick 11 durch den Schlauch 9 auf den Atemaufsatz 3 zu verschiebt, bis eine Luftblase an dem freien Ende des Schlauchs 9 austreten kann. Wie bei den Stimmzungen entsteht hierbei eine Oszillation, die von einem Mikrofon eines Datenverarbeitungsgeräts erfasst und ausgewertet werden kann.

Nach Bedarf kann neben der eigentlichen Atemübung auch eine Aromatherapie oder eine Verneblung von Wirkstoffen in dem Gehäuse 2 erfolgen, so dass die damit versetzte Atemluft von dem Anwender 22 eingeatmet wird. Hierzu ist eine Inhalationsvorrichtung 12 über einen Verbindungsschlauch 13 mit dem Gehäuse 2 so verbunden, dass der Verbindungsschlauch 13 in den Strömungskanal 6 mündet. Neben der Luft, die durch den Schlauch in das Atemübungsgerät 1 dringt, kommt so auch der in der Inhalierkammer 12 vorgehaltene Wirkstoff hinzu, welcher beim Inhalieren durch den Anwender 22 seine Wirkung entfalten kann. Eine solche Maßnahme kann nach Bedarf von der Auswerteeinheit 20 des Datenverarbeitungsgeräts 16 vorgenommen werden, wenn die Ergebnisse früherer Atemübungen oder die Datenlage dies erfordert.

Vorstehend beschrieben ist somit ein Verfahren, bei dem mithilfe eines einfachen und für den Anwender leicht bedienbaren Atemübungsgeräts ein individuelles Training oral, tracheal und/oder nasal durchführbar ist, welches über eine Auswerteeinheit sowohl anleitbar als auch überwachbar ist und aufgrund einer Rückmeldung auswertbar und aufgrund weiterer Umgebungsparameter anpassbar ist.

### BEZUGSZEICHENLISTE

- 1: Atemübungsgerät
- 2: Gehäuse
- 3: Mundstück
- 4: Stimmzungeneinheit
- 5: Stenose
- 6: Strömungskanal
- 7: erste Stimmzunge
- 8: zweite Stimmzunge
- 9: Schlauch
- 10: Rohrleitung
- 11: Knick
- 12: Inhalationsvorrichtung
- 13: Verbindungsschlauch
- 14: Inspirationsvorgang
- 15: Exspirationsvorgang
- 16: Datenverarbeitungsgerät
- 17: Mikrofon
- 18: Bildschirm
- 19: Lautsprecher
- 20: Auswerteeinheit
- 21: Nasenstück
- 22: Anwender

## Patentansprüche

1. Verfahren zur Erfassung an einem Atemübungsgerät (1) durchgeführter Atemübungen, wobei das Atemübungsgerät (1) einen Strömungskanal (6) ausbildet, in dem während der Atemübungen durch eine hierbei eingebrachte Luftströmung ein Strömungsgeräusch erzeugt wird, welches mithilfe eines Mikrofons (17) erfasst und von einer Auswerteeinheit (20) ausgewertet wird,
**dadurch gekennzeichnet, dass** die Auswerteeinheit (20) während der Atemübung ein Atemmuster vorgibt und Intensität und/oder Frequenzverlauf und/oder Dauer und/oder Amplitude des erfassten Strömungsgeräuschs auswertet und, vorzugsweise im Zuge einer Adhärenzmessung, mit dem vorgegebenen Atemmuster vergleicht und mithilfe von Ausgabemitteln der Auswerteeinheit (20) eine Rückmeldung ausgibt.

2. Verfahren gemäß Anspruch 1, **dadurch gekennzeichnet, dass** ein Atemmuster zumindest eine Exhalation umfasst, vorzugsweise zusätzlich zumindest eine Inhalation und/oder wenigstens eine inhalatorische Pause und/oder eine exhalatorische Pause umfasst.

3. Verfahren gemäß einem der Ansprüche 1 oder 2, **dadurch gekennzeichnet, dass** in dem Strömungskanal (6) wenigstens eine Oszillationseinheit, vorzugsweise lösbar, derart aufgenommen ist, dass sie von der Luftströmung durch den Strömungskanal (6) zum akustisch wahrnehmbaren Schwingen angeregt wird.

4. Verfahren gemäß Anspruch 3, **dadurch gekennzeichnet, dass** die Oszillationseinheit auf eine Mundatmung und/oder auf eine Nasenatmung und/oder auf eine tracheale Atmung ausgelegt ist, insbesondere eine Stimmzungeneinheit (4) mit wenigstens einer schwingfähig aufgehängten Stimmzunge (7, 8) und/oder einen Trichter und/oder einen in einen geknickten Verlauf gezwängten Schlauch (9) und/oder ein Kugelventil und/oder ein Klappenventil aufweist.

5. Verfahren gemäß einem der vorhergehenden Ansprüche, **dadurch gekennzeichnet, dass** die Auswerteeinheit ein inspiratorisches Strömungsgeräusch von einem exspiratorischen Strömungsgeräusch unterscheidet und die beiden Strömungsgeräusche für den Vergleich mit dem Atemmuster separat auswertet.

6. Verfahren gemäß einem der vorhergehenden Ansprüche, **dadurch gekennzeichnet, dass** dem Strömungskanal (6) eine veränderliche Stenose (5) zugeordnet ist, deren Öffnungsgrad anhand eines Störgeräuschs und/oder einer Veränderung des Frequenzverlaufs durch die Auswerteeinheit (20) ermittelt wird.

7. Verfahren gemäß einem der vorhergehenden Ansprüche, **dadurch gekennzeichnet, dass** die Auswerteeinheit (20) das Strömungsgeräusch bei einer Atmung durch ein mit dem Atemübungsgerät (1), vorzugsweise lösbar, verbundenes Nasenstück (21), Mundstück (3) oder eine Trachealkanüle anhand von Intensität und/oder Frequenzverlauf und/oder Dauer und/oder Amplitude des erfassten Strömungsgeräuschs unterscheidet.

8. Verfahren gemäß einem der vorhergehenden Ansprüche, **dadurch gekennzeichnet, dass** die Auswerteeinheit (20) einen Ablauf mehrerer Atemzyklen vorgibt und ermittelt, wie viele dieser Atemzyklen innerhalb einer Atemübung ausgeführt werden, wobei vorzugsweise ein Trainingserfolg über einen längeren Zeitraum hinweg erfasst und die Trainingsintensität und -dauer von der Auswerteeinheit an den Fortschritt angepasst wird.

9. Verfahren gemäß einem der vorhergehenden Ansprüche, **dadurch gekennzeichnet, dass** die Auswerteeinheit (20) eingangs einer Atemübung einen Initialschritt vornimmt, bei dem Intensität und/oder Schwingfrequenzverlauf und/oder Dauer und/oder Amplitude der Strömungsgeräusche des Atemübungsgeräts (1) sowie Umgebungsgeräusche erfasst werden, wobei während der nachfolgenden Atemübung ein Bereich um die Intensität und/oder Schwingfrequenzverlauf und/oder Dauer und/oder Amplitude des Strömungsgeräuschs des Atemübungsgeräts (1) mithilfe eines Bandfilters isoliert und Umgebungsgeräusche ausgefiltert werden.

10. Verfahren gemäß Anspruch 9, **dadurch gekennzeichnet, dass** die Auswerteeinheit (20) im Rahmen des Initialschritts empfangene Tonsignale auf das Klangbild eines Ultraschallverneblers oder anderer Medikamentenvernebler prüft.

11. Verfahren gemäß einem der vorhergehenden Ansprüche, **dadurch gekennzeichnet, dass** die Auswerteeinheit (20) dem Atemübungsgerät (1) baueinheitlich zugeordnet ist.

12. Verfahren gemäß einem der Ansprüche 1 bis 10, **dadurch gekennzeichnet, dass** die Auswerteeinheit (20) einem Datenverarbeitungsgerät (16) zugeordnet ist und dem Atemübungsgerät (1) elektronische Kommunikationsmittel zum Datenaustausch mit dem Datenverarbeitungsgerät (16) zugeordnet sind, wobei zumindest Daten zur Erkennung des Typs des Atemübungsgeräts (1) über die elektronischen Kommunikationsmittel durch das Atemübungsgerät (1) an das Datenverarbeitungsgerät (16) übermittelbar sind.

13. Verfahren gemäß Anspruch 12, **dadurch gekennzeichnet, dass** auf dem Datenverarbeitungsgerät (16) Atemübungen mit unterschiedlichen Abläufen, insbesondere hinsichtlich Intensität und/oder Schwingfrequenzverlauf und/oder Dauer und/oder Amplitude der Strömungsgeräusche, Einstellungen und/oder Tonfolgen, gespeichert sind und über die Ausgabemittel der Auswerteeinheit Anweisungen zur Durchführung dieser Atemübungen, vorzugsweise einschließlich einer Einbettung in Musik- und Videodaten zur Begleitung der Atemübungen, bereitgestellt werden.

14. Verfahren gemäß einem der Ansprüche 12 oder 13, **dadurch gekennzeichnet, dass** dem Datenverarbeitungsgerät (20) Mittel zur Dateneingabe und/oder zum Erfassen und/oder zum Abrufen von Daten von einer Datenbank und/oder zum selbsttätigen Vorschlagen oder Festlegen von Daten zugeordnet sind, wobei die Daten
- Anwendergruppierungen und/oder Vorerkrankungen eines Anwenders, insbesondere Obstruktionsgrade der Bronchien bzw. der Nase und/oder Häufigkeiten von Erkältungen und/oder Exazerbationen, Komorbiditäten und/oder Symptomlasten mit und ohne Nies-sowie Hustendetektion,
- und/oder Parameter von stationärer und ambulanter Rehabilitationsmaßnahmen und/oder stationärer und ambulanter therapeutischer und/oder ärztlicher Betreuung oder einer Medikamentierung mit und ohne Nasenspülung eines Anwenders (22),
- und/oder Orts- und/oder Kalenderdaten, Veranstaltungsdaten oder Gesundheitswarnungen und/oder Krankheitsstatistiken öffentlicher Stellen wie beispielsweise in Deutschland das RKI,
- und/oder Positionsdaten, sowie vorzugsweise auch anonymisierte Anwenderdaten anderer Anwender
umfassen.

15. Verfahren gemäß Anspruch 14, **dadurch gekennzeichnet, dass** dem Datenverarbeitungsgerät (16) eine künstliche Intelligenz zugeordnet ist, welche eingegebene und/oder erfasste Daten und/oder Parameter auswertet und Vorschläge für Atemübungen oder andere Daten und/oder Parameter unterbreitet.

16. Verfahren gemäß einem der Ansprüche 12 bis 15, **dadurch gekennzeichnet, dass** die Rückmeldung ein Punktesystem, eine Fortschrittsvisualisierung oder zumindest ein Gamification-Element umfasst.

17. Verfahren gemäß einem der Ansprüche 12 bis 16, **dadurch gekennzeichnet, dass** das Datenverarbeitungsgerät (16) ein Laptop, ein Tablet, ein Smartphone, ein Wearable, ein Lungenfunktionsgerät, ein CPAP, ein Cough-Assist und/oder ein Hustendetektor ist.

18. Verfahren gemäß einem der vorhergehenden Ansprüche, **dadurch gekennzeichnet, dass** die Ausgabemittel einen Bildschirm (18) und/oder einen Lautsprecher (19), etwa einen Kopfhörer, umfassen.

## Geänderte Patentansprüche

### Geänderte Patentansprüche gemäss Regel 137(2) EPÜ.

1. System zur Durchführung von Atemübungen, umfassend ein Atemübungsgerät (1) und eine Auswerteeinheit (20), wobei das Atemübungsgerät (1) einen Strömungskanal (6) zur Erzeugung eines Strömungsgeräuschs während der Atemübungen durch eine hierbei eingebrachte Luftströmung aufweist, wobei ein Mikrofon (17) zur Erfassung des Strömungsgeräuschs vorgesehen ist und die Auswerteeinheit (20) zur Auswertung des Strömungsgeräuschs hergerichtet ist, wobei die Auswerteeinheit (20) dazu hergerichtet ist, während der Atemübung ein Atemmuster vorzugeben und Intensität und/oder Frequenzverlauf und/oder Dauer und/oder Amplitude des erfassten Strömungsgeräuschs auszuwerten und, vorzugsweise im Zuge einer Adhärenzmessung, mit dem vorgegebenen Atemmuster zu vergleichen und mithilfe von Ausgabemitteln der Auswerteeinheit (20) eine Rückmeldung auszugeben,
**dadurch gekennzeichnet, dass** in dem Strömungskanal (6) wenigstens eine Oszillationseinheit, vorzugsweise lösbar, derart aufgenommen ist, dass sie von der Luftströmung durch den Strömungskanal (6) zum akustisch wahrnehmbaren Schwingen angeregt wird.

2. System gemäß Anspruch 1, **dadurch gekennzeichnet, dass** ein Atemmuster zumindest eine Exhalation umfasst, vorzugsweise zusätzlich zumindest eine Inhalation und/oder wenigstens eine inhalatorische Pause und/oder eine exhalatorische Pause umfasst.

3. System gemäß einem der Ansprüche 1 oder 2, **dadurch gekennzeichnet, dass** die Oszillationseinheit auf eine Mundatmung und/oder auf eine Nasenatmung und/oder auf eine tracheale Atmung ausgelegt ist, insbesondere eine Stimmzungeneinheit (4) mit wenigstens einer schwingfähig aufgehängten Stimmzunge (7, 8) und/oder einen Trichter und/oder einen in einen geknickten Verlauf gezwängten Schlauch (9) und/oder ein Kugelventil und/oder ein Klappenventil aufweist.

4. System gemäß einem der vorhergehenden Ansprüche, **dadurch gekennzeichnet, dass** die Auswerteeinheit dazu hergerichtet ist, ein inspiratorisches Strömungsgeräusch von einem exspiratorischen Strömungsgeräusch zu unterscheiden und die beiden Strömungsgeräusche für den Vergleich mit dem Atemmuster separat auszuwerten.

5. System gemäß einem der vorhergehenden Ansprüche, **dadurch gekennzeichnet, dass** dem Strömungskanal (6) eine veränderliche Stenose (5) zugeordnet ist, deren Öffnungsgrad anhand eines Störgeräuschs und/oder einer Veränderung des Frequenzverlaufs durch die Auswerteeinheit (20) ermittelt wird.

6. System gemäß einem der vorhergehenden Ansprüche, **dadurch gekennzeichnet, dass** die Auswerteeinheit (20) dazu hergerichtet ist, das Strömungsgeräusch bei einer Atmung durch ein mit dem Atemübungsgerät (1), vorzugsweise lösbar, verbundenes Nasenstück (21), Mundstück (3) oder eine Trachealkanüle anhand von Intensität und/oder Frequenzverlauf und/oder Dauer und/oder Amplitude des erfassten Strömungsgeräuschs zu unterscheiden.

7. System gemäß einem der vorhergehenden Ansprüche, **dadurch gekennzeichnet, dass** die Auswerteeinheit (20) dazu hergerichtet ist, einen Ablauf mehrerer Atemzyklen vorzugeben und zu ermitteln, wie viele dieser Atemzyklen innerhalb einer Atemübung ausgeführt werden, wobei sie vorzugsweise dazu hergerichtet ist, einen Trainingserfolg über einen längeren Zeitraum hinweg zu erfassen und die Trainingsintensität und Trainingsdauer von der Auswerteeinheit an den Fortschritt anzupassen.

8. System gemäß einem der vorhergehenden Ansprüche, **dadurch gekennzeichnet, dass** die Auswerteeinheit (20) dazu hergerichtet ist, eingangs einer Atemübung einen Initialschritt vorzunehmen, bei dem Intensität und/oder Schwingfrequenzverlauf und/oder Dauer und/oder Amplitude der Strömungsgeräusche des Atemübungsgeräts (1) sowie Umgebungsgeräusche erfasst werden, wobei während der nachfolgenden Atemübung ein Bereich um die Intensität und/oder Schwingfrequenzverlauf und/oder Dauer und/oder Amplitude des Strömungsgeräuschs des Atemübungsgeräts (1) mithilfe eines Bandfilters isoliert und Umgebungsgeräusche ausgefiltert werden.

9. System gemäß Anspruch 8, **dadurch gekennzeichnet, dass** die Auswerteeinheit (20) dazu hergerichtet ist, im Rahmen des Initialschritts empfangene Tonsignale auf das Klangbild eines Ultraschallverneblers oder anderer Medikamentenvernebler zu prüfen.

10. System gemäß einem der vorhergehenden Ansprüche, **dadurch gekennzeichnet, dass** die Auswerteeinheit (20) dem Atemübungsgerät (1) baueinheitlich zugeordnet ist.

11. System gemäß einem der Ansprüche 1 bis 9, **dadurch gekennzeichnet, dass** die Auswerteeinheit (20) einem Datenverarbeitungsgerät (16) zugeordnet ist und dem Atemübungsgerät (1) elektronische Kommunikationsmittel zum Datenaustausch mit dem Datenverarbeitungsgerät (16) zugeordnet sind, wobei zumindest Daten zur Erkennung des Typs des Atemübungsgeräts (1) über die elektronischen Kommunikationsmittel durch das Atemübungsgerät (1) an das Datenverarbeitungsgerät (16) übermittelbar sind.

12. System gemäß Anspruch 11, **dadurch gekennzeichnet, dass** auf dem Datenverarbeitungsgerät (16) Atemübungen mit unterschiedlichen Abläufen, insbesondere hinsichtlich Intensität und/oder Schwingfrequenzverlauf und/oder Dauer und/oder Amplitude der Strömungsgeräusche, Einstellungen und/oder Tonfolgen, gespeichert sind und die Auswerteeinheit dazu hergerichtet ist, über die ihr zugeordneten Ausgabemittel Anweisungen zur Durchführung dieser Atemübungen, vorzugsweise einschließlich einer Einbettung in Musik- und Videodaten zur Begleitung der Atemübungen, bereitzustellen.

13. System gemäß einem der Ansprüche 11 oder 12, **dadurch gekennzeichnet, dass** dem Datenverarbeitungsgerät (20) Mittel zur Dateneingabe und/oder zum Erfassen und/oder zum Abrufen von Daten von einer Datenbank und/oder zum selbsttätigen Vorschlagen oder Festlegen von Daten zugeordnet sind, wobei die Daten
- Anwendergruppierungen und/oder Vorerkrankungen eines Anwenders, insbesondere Obstruktionsgrade der Bronchien bzw. der Nase und/oder Häufigkeiten von Erkältungen und/oder Exazerbationen, Komorbiditäten und/oder Symptomlasten mit und ohne Nies-sowie Hustendetektion,
- und/oder Parameter von stationärer und ambulanter Rehabilitationsmaßnahmen und/oder stationärer und ambulanter therapeutischer und/oder ärztlicher Betreuung oder einer Medikamentierung mit und ohne Nasenspülung eines Anwenders (22),
- und/oder Orts- und/oder Kalenderdaten, Veranstaltungsdaten oder Gesundheitswarnungen und/oder Krankheitsstatistiken öffentlicher Stellen wie beispielsweise in Deutschland das RKI,
- und/oder Positionsdaten, sowie vorzugsweise auch anonymisierte Anwenderdaten anderer Anwender
umfassen.

14. System gemäß Anspruch 13, **dadurch gekennzeichnet, dass** dem Datenverarbeitungsgerät (16) eine künstliche Intelligenz zugeordnet ist, welche dazu hergerichtet ist, eingegebene und/oder erfasste Daten und/oder Parameter auszuwerten und Vorschläge für Atemübungen oder andere Daten und/oder Parameter zu unterbreiten.

15. System gemäß einem der Ansprüche 11 bis 14, **dadurch gekennzeichnet, dass** die Rückmeldung ein Punktesystem, eine Fortschrittsvisualisierung oder zumindest ein Gamification-Element umfasst.

16. System gemäß einem der Ansprüche 11 bis 15, **dadurch gekennzeichnet, dass** das Datenverarbeitungsgerät (16) ein Laptop, ein Tablet, ein Smartphone, ein Wearable, ein Lungenfunktionsgerät, ein CPAP, ein Cough-Assist und/oder ein Hustendetektor ist.

17. System gemäß einem der vorhergehenden Ansprüche, **dadurch gekennzeichnet, dass** die Ausgabemittel einen Bildschirm (18) und/oder einen Lautsprecher (19), etwa einen Kopfhörer, umfassen.
